# EUROPÄISCHE PATENTANMELDUNG

(11) **EP 0 600 140 A1**
(43) Veröffentlichungstag der Anmeldung: **08.06.1994**
(21) Anmeldenummer: 92810959.4
(22) Anmeldetag: 04.12.1992
(51) Int. Cl.: A61F 2/00, A61F 2/06, A61L 2/26

(54) **Behälter zum Verpacken einer hohlkörperartigen Endoprothese**

(71) Anmelder: SULZER Medizinaltechnik AG, CH-8404 Winterthur (CH)
(72) Erfinder: Bittmann, Peter, Dr., CH-8057 Zürich (CH); Tanner, Fredy, CH-9500 Wil (CH); Müller-Glauser, Werner, Dr., CH-8542 Wiesendangen (CH)
(74) Vertreter: Hammer, Bruno, Dr.

(57) **Zusammenfassung**

Der Behälter zum Verpacken von hohlkörperartigen Endoprothesen (8) weist zwei keimundurchlässige Hüllen (1, 2) auf. Erfindungsgemäss besteht die innere Hülle (1) aus einer wasserfesten Wand (10, 11) und diversen Anschlussstellen (40, 50, 60). Mindestens zwei der Anschlussstellen (40) sind als Zugang in den Hohlraum der Prothese (8) und eine als Ablauf (50) für Flüssigkeiten zum Behandeln der Prothese vorgesehen. Die äussere Hülle (2) weist Durchgangsstücke (42, 52, 62) auf, die den Anschlussstellen der inneren Hülle zugeordnet sind. Die beiden Hüllen sind zumindest bereichsweise gasdurchlässig. Der Behälter hat erfindungsgemäss neben der Schutzfunktion einen zweiten Verwendungszweck: beispielsweise im Falle einer Hybridprothese für den Gefässersatz die Beladung der Prothese mit patienteneigenen Endothelzellen im Behälter durchzuführen.

## Beschreibung

Die Erfindung bezieht sich auf einen Behälter mit zwei keimundurchlässigen Hüllen zum Verpacken einer hohlkörperartigen Endoprothese, insbesondere einer sogenannten Hybridprothese, die aus einer künstlichen, alloplastischen Komponente einerseits und aus lebenden körpereigenen Zellen, Zellverbänden oder Gewebeteilen des Patienten andererseits besteht. Derartige Prothesen sind beispielsweise schlauchförmige Trägerstrukturen aus Kunststoff, die mit patienteneigenen Endothelzellen beladen werden und die als Gefässersatz dienen (siehe z.B. EP-PS 0 320 441 = P.6146). Ein anderes Beispiel sind Endoprothesen, die als Organersatz Vorgesehen sind und die Trägerstrukturen für lebende Zellen und/oder andere Körperbestandteile bilden, wobei diese Prothesen zwar hohlkörperartig aber nicht notwendigerweise schlauchförmig sind und beispielsweise in ihrem Hohlraum zusätzlich eine schwamm- oder netzartige Trägerstruktur aufweisen (siehe z.B. US-PS 4 963 489 (Naughton et al.) oder DE-OS 39 36 568 (Schmidt)).

Beim Einsatz von Implantaten im menschlichen oder tierischen Körper muss in jedem Augenblick Keimfreiheit gewährleistet sein. Denn Keime, die das Implantat kontaminieren, können vom körpereigenen Abwehrsystem nur schwer oder gar nicht erreicht und deshalb auch nicht bekämpft werden. Ein sich ausbildender Infektionsherd hätte unvermeidlich eine Reoperation zur Folge.

Je nach Verweilort und Verweilzeit eines Implantats sind die Anforderungen an die das Implantat schützende Verpackung unterschiedlich. Kurzzeitimplantate, die in natürliche Höhlen oder Kanäle des Körpers eingesetzt werden, sind meist nur durch eine einzige keimundurchlässige Hülle geschützt. Implantate, die innig mit dem menschlichen Gewebe in Berührung kommen, beispielsweise Prothesen für den Gefässersatz, werden üblicherweise durch zwei Keimbarrieren vor einer Kontamination geschützt: durch eine Primärverpackung oder Produktverpackung und durch eine Sekundärverpackung oder Schutzverpackung. Dank diesem doppelten Schutz kann nach Entfernung der äusseren Hülle (Schutzverpackung) die allseitig keimfreie innere Hülle (Produktverpackung) ohne Bedenken im sterilen Operationsfeld verwendet werden.

Es ist Aufgabe der Erfindung, für eine hohlkörperartige Endoprothese, insbesondere eine Hybridprothese, einen Behälter zu schaffen, der einerseits als Verpackung dient, für welche die Sterilität des Implantats in jedem Augenblick gewahrt ist, und der andererseits zusätzlich das Beladen der Trägerstruktur, d.h. der alloplastischen Komponente, mit lebenden Zellen, Zellverbänden oder Gewebeteilen des Patienten ermöglicht. Diese Aufgabe lässt sich erfindungsgemäss mit dem im Anspruch 1 gekennzeichneten Behälter lösen.

Im Hinblick auf die im folgenden beschriebene Behandlung der Prothese sei noch darauf hingewiesen, dass die Prothesenwand porös und daher für Gas und Wasser durchlässig ist.

Die Herstellung der Prothese wie auch die des erfindungsgemässen Behälters braucht nicht unter sterilen Bedingungen durchgeführt zu werden. Denn nach dem Einpacken der Prothese in die Doppelhülle des Behälters (wobei zum Einpacken selbstverständlich auch das Anschliessen der Prothese an die Anschlussstellen der inneren Hülle gehört) kann eine Sterilisation durchgeführt werden.

Die Wahl der Sterilisation richtet sich nach der Beschaffenheit und den Materialien der Trägerstruktur. Mit Vorteil wird man eine Dampfsterilisation oder eine Sterilisation mittels Gammastrahlen wählen, wenn die Prothese und die für die Verpackung verwendeten Materialien gegenüber einer solchen Behandlung beständig sind. Andernfalls wird die Sterilisation mittels Ethylenoxid durchgeführt (sogenannte Gassterilisation).

Bei der Gassterilisation lässt man über mindestens eine der Prothesen-Anschlussstellen - und durch das entsprechende Durchgangsstück in der äusseren Hülle - das Ethylenoxid in den Hohlraum der Prothese einströmen. Dank der Gasdurchlässigkeit der beiden Hüllen sowie der Prothesenwand kann sich das Gas überall innerhalb des Behälters verbreiten und die sterilisierende Wirkung entfalten.

Weil die Prothese mit lebendem Gewebe beschickt werden soll, ist es nach einer Gassterilisation unerlässlich, dass das Ethylenoxid vor der Beladung rückstandfrei aus dem Implantat entfernt wird. Dies geschieht - ebenfalls dank der Gasdurchlässigkeit der beiden Hüllen und der Prothesenwand - durch mehrfaches Belüften mit steriler Luft (Belüftungszyklen).

Nach der Sterilisation ist die Prothese für weitere Präparationsschritte bereit, bei welchen die Prothese mittels Flüssigkeiten behandelt wird. Diese Verfahrensschritte werden anhand des Beispiels einer Gefässprothese beschrieben; ein entsprechendes Verfahren lässt sich auch auf die Behandlung von hohlkörperartigen Organprothesen übertragen. Das Beispiel betrifft eine Hybridprothese, die durch Beladen einer schlauchförmigen, porösen Kunststoffwand mit patienteneigenen Endothelzellen hergestellt wird und die eine partielle Abdichtung der Wand durch Gelatine aufweist. (Die Bezeichnung "Prothese" bezieht sich auf die Kunststoffwand, also auf die Trägerstruktur, die mit Zellen beladbar ist; unter "Hybridprothese" wird die beladene Prothese verstanden.) Die Prothese des Beispiels wird in getrocknetem Zustand in den erfindungsgemässen Behälter eingeschlossen und sterilisiert. Auf diese Weise verpackt kann die Prothese bis zur Verwendung gelagert werden.

Das Beladen der Hybridprothese, d.h. das Gewinnen der Patienten-Zellen sowie das Beschichten des Kunststoffschlauches mit diesen Zellen, muss während der Operation, bei welcher der Gefässersatz erfolgt, ausgeführt werden. Für die Präparation der Hybridprothese steht höchstens eine Stunde zur Verfügung. Die verschiedenen, dazu notwendigen Schritte, beispielsweise das mechanische Schneiden des patienteneigenen Ausgangsgewebes sowie der anschliessende enzymatische Aufschluss werden in Form eines geschlossenen Prozesses in einer Maschine durchgeführt, die hier nicht beschrieben wird. Der erfindungsgemässe Behälter ist nicht nur Verpackung und Schutz der Prothese, sondern gleichzeitig auch integrierender Bestandteil dieser Maschine.

Bei der Präparation der Hybridprothese für die Operation sind folgende Arbeitsschritte auszuführen:
1. Die beiden Anschlussstellen der Prothese, ein Überlauf (falls vorhanden) und der Ablauf werden mittels Schläuchen an die genannte Maschine angeschlossen.
2. Mit Wasser wird die Luft aus dem Hohlraum der Prothese verdrängt.
3. Eine Anschlussstelle der Prothese wird verschlossen; durch die andere Anschlussstelle wird Wasser unter Druck zugeführt. Dabei wird Wasser durch die Prothesenwand durchgepresst. Bei geschlossenem Ablauf füllt sich die innere Hülle mit Wasser. Die verdrängte Luft entweicht durch die luftdurchlässigen Bereiche der Hüllenwände und gegebenenfalls durch den Überlauf. Die Prothese befindet sich schliesslich vollständig unter Wasser; ein Niederhalter verhindert ein Aufschwimmen der Prothese.
4. Während einer Wartezeit von fünf bis zehn Minuten quillt die Gelatine in der Prothesenwand. (Sogenanntes "Äquilibrieren" der Prothese. Falls eine Prothese ohne Gelatineabdichtung beladen wird, fällt dieser Schritt weg.)
5. Eine Suspension der Endothelzellen wird in den Hohlraum der Prothese gepumpt und durch die Prothesenwand durchgepresst. Dabei lagern sich die Zellen auf und in der Wand, d.h. auf der Trägerstruktur, ab. Damit die Beladung gleichmässig erfolgt, muss die Prothese weitgehend horizontal ausgerichtet sein.
6. Der Ablauf wird geöffnet, sodass sich die innere Hülle entleert.
7. Nach der Entleerung der inneren Hülle wird die Prothese "radial", d.h. bei einem Durchfluss durch die Prothesenwand, gespült. Der Ablauf wird wieder geschlossen, sodass sich die innere Hülle mit der Spülflüssigkeit füllt.
8. Nach erneutem Entleeren der inneren Hülle wird das radiale Spülen wiederholt. Zweck des Spülens ist es, Stoffe, die in der Zellsuspension gelöst sind (insbesondere das zum Aufschluss des Gewebes verwendete Enzym), möglichst weitgehend zu entfernen.
9. Schliesslich wird die Prothese "axial" gespült, indem man die Spülflüssigkeit aus der Zweiten Anschlussstelle austreten lässt.

Nach dieser Präparation der Prothese wird die äussere Hülle (Schutzhülle) geöffnet. Aus der geöffneten Hülle kann nun die beladene Prothese, die in die allseitig keimfreie Produktpackung eingehüllt ist, entnommen und in den sterilen Operationsbereich gebracht werden.

Die abhängigen Ansprüche betreffen vorteilhafte Ausführungsformen des erfindungsgemässen Behälters. Die Ansprüche 11 und 12 beziehen sich auf Anwendungen des Behälters.

Die Erfindung wird nachfolgend anhand von Zeichnungen näher erläutert. Es zeigen:
- Fig. 1: eine Explosionsdarstellung mit drei Teilen einer ersten Ausführungsform des erfindungsgemässen Behälters,
- Fig. 2: einen vertikalen Querschnitt durch den Behälter der Fig.1 (ohne Prothese), nach Linie II-II in Fig.3,
- Fig. 3: einen horizontalen Querschnitt durch die innere Hülle desselben Behälters (mit Prothese), nach Linie III-III in Fig.2,
- Fig. 4: eine untere Ansicht einer Schale, welche die innere Hülle für eine zweite Ausführungsform bildet,
- Fig. 5: ein Bauteil einer mit einer Membran verschlossenen Anschlussstelle für die Prothese,
- Fig. 6: ein Durchgangsstück mit Membranabschluss,
- Fig. 7: eine Hohlnadel mit Schlauch und
- Fig. 8: ein kurzes Stück einer Gefässprothese.

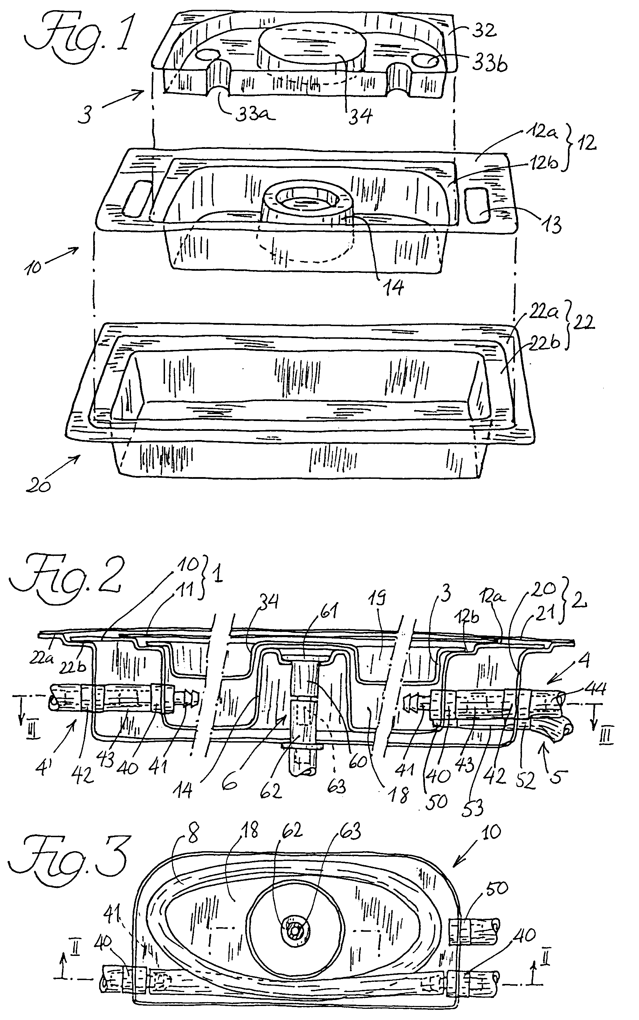

Die in den Figuren 1 und 2 dargestellte innere Hülle 1 (Produktverpackung) besteht aus einer Schale 10 und einer Deckfolie 11, die äussere Hülle 2 (Schutzverpackung) aus einer Schale 20 und einer Deckfolie 21. Die Deckfolien 11 und 21 sind mit den Rändern 12 bzw. 22 der entsprechenden Schalen 10 bzw. 20 und zwar mit den Randzonen 12a bzw. 22a abschälbar verbunden. Der Rand 22 der äusseren Schale 20 weist eine Vertiefung 22b auf, in die der Rand 12 der inneren Schale 21 eingelegt wird. Die innere Schale 21 weist eine entsprechende Vertiefung für einen Niederhalter 3 auf.

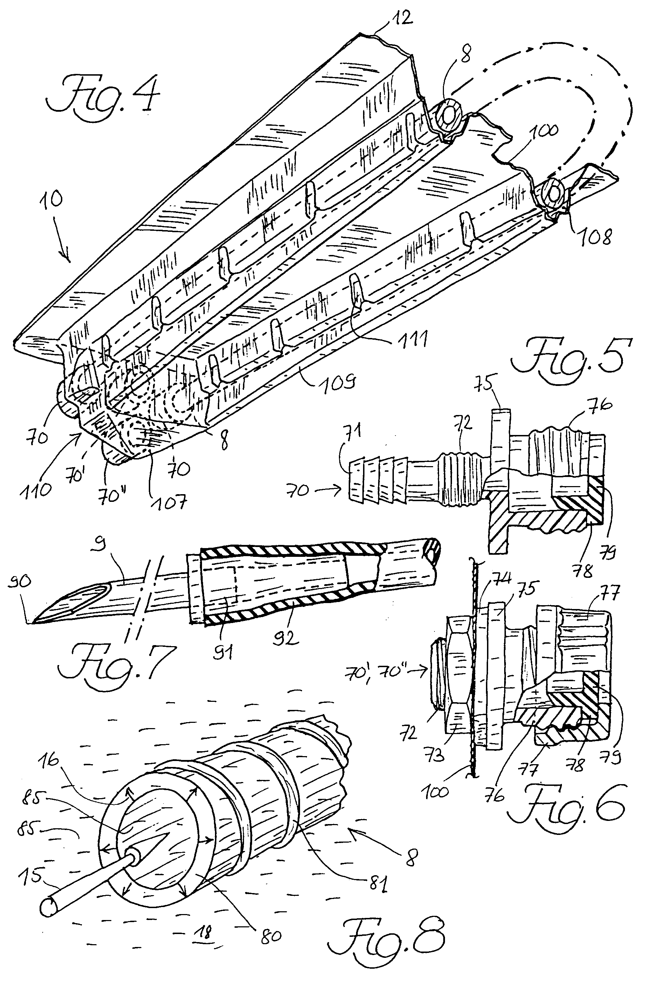

Aus der inneren Hülle 1 führen mehrteilige Verbindungsstücke 4, 4', 5 und 6 zur Aussenseite der äusseren Hülle 2. (In der Fig.1 sind der Einfachheit halber weder diese Verbindungsstücke noch Durchbrüche, die für sie vorgesehen sind, dargestellt.) Das Verbindungsstück 4 besteht aus einer Anschlussstelle 40 mit einem Nippel 41 für die Prothese 8 (Fig.3), einem Durchgangsstück 42 in der äusseren Hülle 2 und einem verbindenden Schlauchstück 43. Am Durchgangsstück 42 ist eine Schlauch 44 angeschlossen. (Beim Lagern der verpackten Prothese ist statt des Schlauchs 44 eine Verschlusskappe vorgesehen, die eine Kontamination des Behälterinneren verhindert.) Das andere Ende der Prothese 8 ist an ein zweites Verbindungsstück 4' angeschlossen, das den gleichen Aufbau wie das Verbindungsstück 4 hat. Ein ähnliches Verbindungsstück 5 ist für einen Ablauf 50 vorgesehen. In der Mitte der inneren Schale 10 bildet ein zylindrisches Wandstück 14 zusammen mit einem Verbindungsstück 6 einen Überlauf, der sich aus einer Anschlussstelle 60 mit einer Überlaufmündung 61, einem Durchgangsstück 62 in der äusseren Hülle 2 und einer Steckverbindung 63 aufbaut.

Der Niederhalter 3 hat die Aufgabe, die Prothese bei den oben beschriebenen Präparierungsschritten am Aufschwimmen zu hindern. Seitliche Kanäle 33a und Durchbrüche 33b sorgen für eine gute Durchlässigkeit für Gas und Flüssigkeit zwischen der Prothesenkammer 18 (Fig.2) und dem Raum 19 zwischen Niederhalter 3 und Deckfolie 11.

Die unteren Teile der beiden Hüllen 1 und 2 sind geformte Schalen 10 bzw. 20, die mit Vorteil durch Warmformen aus Thermoplastfolien hergestellt werden. Die Deckfolien 11 und 21 sind gasdurchlässig und bestehen mit Vorteil aus einem reissfesten, papierartigen Material, beispielsweise aus "Tyvek" (Handelsname). Die Deckfolien können mit den Schalen durch Schweissen verbunden werden, wobei eine leicht trennbare Verbindung vorzusehen ist, die ein Abschälen der Deckfolien ermöglicht.

Damit nach dem Öffnen der äusseren Hülle 2 die innere Hülle 1 entnommen werden kann, müssen die Verbindungen zwischen den Anschlussstellen 40, 50 der Hülle 1 und den Durchgangsstücken 42, 52 (Fig.2) der Hülle 2 durchtrennt werden. Zu diesem Zweck sind im Rand 12 der Schale 10 Öffnungen 13 vorgesehen, sodass durch diese Öffnungen 13 die Schlauchstücke 43, 53 mittels einer Schere durchschnitten werden können. Die Verbindung des Überlaufs 6 wird durch Herausziehen der Steckverbindung 63 getrennt.

Bei der Ausführungsform der Fig.4 sind die Anschlussstellen 70 (Prothesenanschlüsse), 70'(Überlauf) und 70'' (Ablauf) auf einer Seite 110 der Schale 10 angeordnet. Bezüglich der erwähnten Maschine, mit der die Beladung der Prothese vorgenommen wird, ist diese Anordnung gegenüber jener der ersten Ausführungsform zweckmässiger, da der Anschluss an die Maschine mit einer Art Stecker - analog zu einem Elektrostecker - herstellbar ist. Die Prothese 8 liegt in einem U-förmigen, horizontalen Kanal 108, der durch einen nicht dargestellten Niederhalter überdacht wird. Unterhalb des Kanals 108 sind zwei Ablaufrinnen 109 vorgesehen, die in einen Sumpf 107 beim Ablauf 70'' führen. Rippen 111 des Kanals 108 dienen dazu, dass die Prothese 8 nur bei diesen Rippen 111 mit der Wand 100 in Kontakt kommen.

Die Anschlussstellen 70, 70' und 70'' können wie beim ersten Ausführungsbeispiel über Schlauchstücke mit Durchgangsstücken der äusseren (nicht dargestellten) Hülle verbunden sein. Es ist aber möglich, für diese Anschlussstellen bzw. Durchgangsstücke die in den Figuren 5 und 6 gezeigten Bauteile zu verwenden, bei welchen die Verbindung mittels einer Hohlnadel 9 (Fig.7) hergestellt wird:

Die Anschlussstelle 70 für die Prothese besteht aus folgenden Teilen (siehe Fig.5, Fig.6): Anschlussnippel 71 für die Prothese; Gewinde 72 für eine Befestigungsmutter 73 (für die Befestigung an der Wand 100 der Schale 10); Dichtungsring 74; Flansch 75; Membranhalter 76 mit Überwurfmutter 77 und Membranpfropfen 78, Membran 79 (aus einem elastomeren Material). Die Anschlussstellen 70' und 70'' für den Überlauf bzw. Auslauf (Fig.6) unterscheiden sich von den Prothesen-Anschlussstellen 70 im wesentlichen nur durch den fehlenden Nippel 71. Das Bauteil der Fig.6 eignet sich auch als Durchgangsstück der äusseren Hülle. Durchgangsstück und zugeordnete Anschlussstelle der inneren Hülle 10 werden fluchtend angeordnet, sodass mit einer Hohlnadel 9 (Fig.7) die Verbindung hergestellt werden kann. Die Hohlnadel 9 weist eine scharfe Spitze 90 zum Durchstechen der beiden Membranen 79 auf; und sie ist über ein Übergangsstück 91 mit einem Schlauch 92 verbunden. Die Anschlüsse der Prothese müssen selbstverständlich aus zellfreundlichen Werkstoffen hergestellt sein.

Die zweite Ausführungsform des erfindungsgemässen Behälters hat gegenüber der ersten Ausführungsform zwei wichtige Vorteile, die sich auf die Präparierungsschritte 3 (Fluten der inneren Hülle) sowie 7 und 8 (Spülen) beziehen: 1. Der Kanal 108 hat ein bedeutend kleineres Volumen als die Prothesenkammer 18. - 2. Die Ablaufrinnen 109 und der Sumpf 107 ermöglichen eine bessere Entleerung. Dank dieser Vorteile wird weniger Flüssigkeit für die Behandlung der Prothese benötigt. Der Präparierungsschritt 3 dauert weniger lang und die Schritte 7 und 8 erfolgen gründlicher.

Fig.8 dient zur ergänzenden Illustration der Präparierungsschritte 3, 5, 7 und 8. Der in der Kammer 18 horizontal liegenden Prothese 8, mit der porösen Wand 80 und einem wendelförmigen Stützdraht 81, wird eine Flüssigkeit 85 axial (Pfeil 15) zugeführt. Aus dem Hohlraum 82 der Prothese 8 strömt die Flüssigkeit 85 radial (Pfeile 16) in die Umgebung. Da die Prothese 8 vollständig von der Flüssigkeit 85 umgeben ist, ergibt sich ein über den Umfang weitgehend konstanter radialer Fluss. Daher erfolgt im Fall, dass die Flüssigkeit 85 die Zellsuspension ist, eine gleichmässige Zellbeladung der Prothesenwand 80, sowohl in Umfangs- als auch in Längsrichtung.

## Patentansprüche

1. Behälter mit zwei keimundurchlässigen Hüllen (1, 2) zum Verpacken von hohlkörperartigen Endoprothesen (8), dadurch gekennzeichnet dass die innere Hülle (1) aus einer wasserfesten Wand (10, 11) und diversen Anschlussstellen (40, 50, 60) besteht, dass mindestens zwei der Anschlussstellen (40) als Zugang in den Hohlraum der Prothese sowie eine als Ablauf (50) für Flüssigkeiten zum Behandeln der Prothese vorgesehen sind, dass die äussere Hülle (2) Durchgangsstücke (42, 52, 62) aufweist, die den Anschlussstellen der inneren Hülle zugeordnet sind, und dass beide Hüllen zumindest bereichsweise gasdurchlässig sind.

2. Behälter nach Anspruch 1, dadurch gekennzeichnet, dass die beiden Hüllen (1, 2) je aus einer geformten Schale (10, 20), aus einer gasdurchlässigen Deckfolie (11, 21) und aus in die Schale eingesetzten Bauteilen der Anschlussstellen (40, 50, 60) bzw. Durchgangsstücke (42, 52, 62) bestehen.

3. Behälter nach Anspruch 2, dadurch gekennzeichnet, dass die Deckfolien (11, 21) abschälbar mit den Schalen (10, 20) verbunden sind.

4. Behälter nach einem der Ansprüchen 2 oder 3, dadurch gekennzeichnet, dass die Schalen (10, 20) durch Warmformen aus einem folienförmigen Thermoplasten hergestellt sind.

5. Behälter nach einem der Ansprüchen 1 bis 4, dadurch gekennzeichnet, dass die für die Prothese (8) vorgesehenen Anschlussstellen (40) sowie die Ablaufstelle (50) über durchschneidbare Schlauchstücke (43, 53) mit den zugeordneten Durchtrittsstücken (42, 52) der äusseren Hülle (2) verbunden sind und dass eine Überlaufstelle (60) über eine Steckverbindung (63) mit einem zugeordneten Durchtrittsstück (62) der äusseren Hülle zusammengeschlossen ist.

6. Behälter nach einem der Ansprüchen 1 bis 5, dadurch gekennzeichnet, dass die Anschlussstellen (70, 70', 70'') der inneren Hülle (1) und die Durchtrittsstücke der äusseren Hülle Membranabschlüsse (79) aus einem elastomeren Material aufweisen und dass die Verbindungen zwischen den Durchtrittsstücken und deren zugeordneten Anschlussstellen mittels Hohlnadeln (9) herstellbar sind.

7. Behälter nach einem der Ansprüchen 1 bis 6, dadurch gekennzeichnet, dass innerhalb der inneren Hülle (1) ein Niederhalter (3) für die Prothese (8) vorgesehen ist.

8. Behälter nach einem der Ansprüchen 1 bis 7, dadurch gekennzeichnet, dass die Anschlussstellen (70, 70', 70'') der inneren Hülle (1) bzw. Durchtrittsstücke der äusseren Hülle an der gleichen Seite des Behälters (110) angeordnet sind.

9. Behälter nach einem der Ansprüchen 1 bis 8, dadurch gekennzeichnet, dass die innere Hülle (1) einen Sumpf (107) aufweist, an dem der Ablauf (70'') angeschlossen ist.

10. Behälter nach Anspruch 9, dadurch gekennzeichnet, dass in der inneren Hülle (1) Rinnen (109), die zum Sumpf (107) führen, vorgesehen sind.

11. Verwendung des Behälters gemäss einem der Ansprüche 1 bis 10, dadurch gekennzeichnet, dass die Prothese (8) im Behälter mit patienteneigenen Körperbestandteilen beladen wird.

12. Verwendung des Behälters nach Anspruch 11, dadurch gekennzeichnet, dass die Prothese (8) eine Gefässprothese ist und dass diese im Behälter mit patienteneigenen Endothelzellen beladen wird.
